# FASCICULE DE BREVET EUROPEEN

(11) **EP 0 754 014 B1**
(45) Date de publication et mention de la délivrance du brevet: **05.08.1998**
(21) Numéro de dépôt: 95915229.9
(22) Date de dépôt: 30.03.1995
(51) Int. Cl.: A61B 17/78, A61B 17/74

(54) **DISPOSITIF D'ENCLOUAGE CENTROMEDULLAIRE NOTAMMENT POUR FRACTURES SOUS CERVICALES DU FEMUR**
INTRAMEDULLÄRNAGEL, INSBESONDERE FÜR SCHENKELHALSFRAKTUREN
CENTROMEDULLARY NAILING DEVICE, IN PARTICULAR FOR SUBCERVICAL FRACTURES OF THE THIGH BONE

(30) Priorité: 01.04.1994 FR 9404401
(43) Date de publication de la demande: 22.01.1997
(73) Titulaire: MEDINOV S.A., F-42300 Roanne (FR)
(72) Inventeur: GRAMMONT, Paul, F-21000 Dijon (FR)
(74) Mandataire: Moncheny, Michel
(86) Numéro de dépôt international: FR9500405
(87) Numéro de publication internationale: WO9526688

(56) Documents cités:
- EP-A- 0 217 317
- EP-A- 0 382 256
- FR-A- 2 501 033
- US-A- 4 827 917

## Description

L'invention se rattache au secteur technique des implants d'ostéosynthèse de l'extrémité du fémur notamment.

Pour réduire ce type de fracture, plusieurs solutions peuvent être employées.

On peut utiliser par exemple, une vis céphalique mise en place par un ancillaire approprié au niveau de la tête du fémur. Cette vis peut être engagée dans un clou centro-médullaire ou bien dans l'alésage d'un fût cylindrique, formé angulairement à partir d'une plaque d'appui diaphysaire fixée sur le fémur. Toute la charge du patient est répartie sur un seul point constitué par l'extrémité de la vis. Il en résulte une instabilité en rotation et un écrasement de la tête fémorale sur l'extrémité de la vis.

On peut citer par exemple le document FR-A-2501033 et, l'enseignement du brevet US 4827917. Ce brevet décrit un dispositif pour le traitement des fractures sous cervicales du fémur, sous forme d'un clou centro-médullaire dont la tête est percée de deux trous d'axes parallèles et situé dans le plan de symétrie du clou. Ces trous recoivent des broches dirigées en direction de la tête du fémur. Les broches sont donc parallèles entre elles et situées dans un même plan.

On connaît également des techniques d'enclouage utilisant plusieurs clous déformables élastiquement, pour être engagés au niveau du col et de la diaphyse du fémur.

Le problème que se propose de résoudre l'invention, est de proposer une autre technique d'ostéosynthèse combinant un système souple afin de faire consolider la fracture sous un effet de mise sous compression du foyer de fracture et un système résistant, afin d'encaisser les efforts de flexion, de l'ensemble tête et diaphyse fémorales.

Pour résoudre un tel problème, il a été conçu et mis au point un dispositif conforme aux caracteristiques de la revendication 1.

L'invention est exposée, ci-après plus en détail à l'aide des dessins annexés, montrant le dispositif d'enclouage comprenant à titre indicatif, nullement limitatif, trois broches et dans lesquels :
La figure 1 est une vue de face du dispositif d'enclouage selon l'invention.
La figure 2 est une vue de dessus correspondant à la figure 1.
La figure 3 est une vue en coupe transversale considérée selon la ligne 3.3 de la figure 2.
La figure 4 est une vue en coupe transversale considérée selon la ligne 4.4 de la figure 2.
La figure 5 est une vue en coupe transversale considérée selon la ligne 5.5 de la figure 2.
La figure 6 est une vue montrant l'ostéosynthèse d'une fracture du col fémoral au moyen du dispositif selon l'invention.
La figure 7 est une vue de côté correspondant à la figure 6.

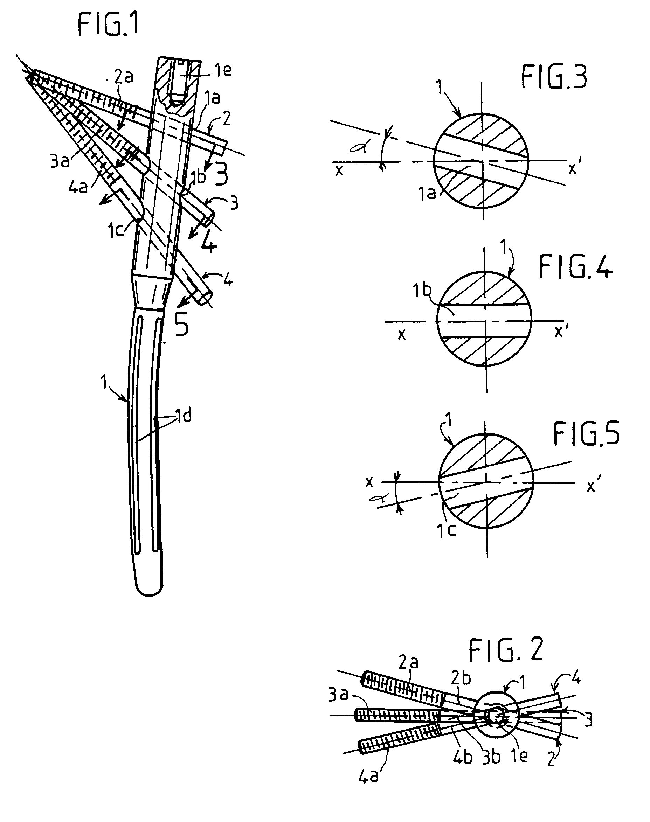

Comme le montre la figure 1, le dispositif comprend un clou centro-médullaire (1), de tout type connu et approprié, et présentant, dans le plan frontal, un léger rayon de courbure pour correspondre au profil anatomique du fémur. Le clou (1) est conformé pour recevoir une série de broches (2) (3) et (4).

Dans ce but, le clou (1) présente trois trous débouchants (1a) (1b) (1c) dans lesquels peuvent être engagées les broches (2) (3) et (4) impactées dans la corticale de l'os.

Selon l'invention, les trous (1a) (1b) (1c) sont orientés angulairement dans un plan vertical et dans un plan horizontal pour que les extrémités (2a) (3a) (4a) des broches (2) (3) et (4) soient alignées sur le diamètre antéro-postérieur de la tête fémorale, ce qui correspond au plan de charge du patient. Avantageusement, les extrémités des broches (2) (3) (4) sont régulièrement réparties sur toute la longueur du diamètre.

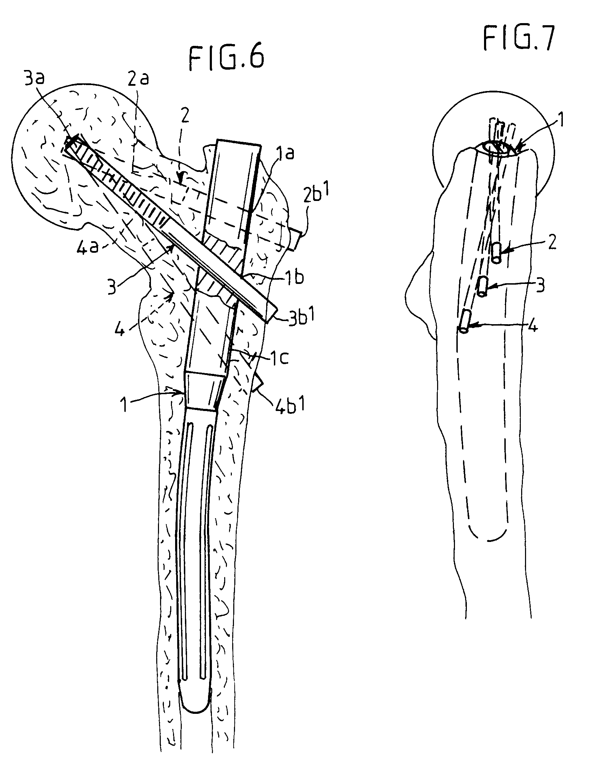

En projection, dans le plan frontal, les trous (1a) (1b) et (1c) sont orientés pour que leurs axes convergent en direction du centre de la tête fémorale (figures 1 et 6).

Par exemple, dans le cas où le cou présente trois broches (2) (3) et (4), dans le plan horizontal, les trous (1a) (1b) (1c) sont orientés angulairement dans les conditions suivantes :
- l'axe du trou supérieur (1a) est orienté d'une valeur angulaire (α) d'environ 15° vers l'avant, par rapport à l'axe (XX') du trou médian (1b) (figure 3),
- l'axe (XX') du trou médian (1b) est orienté très sensiblement perpendiculaire à l'axe général du clou (figure 4),
- l'axe du trou inférieur (1c) est orienté d'une valeur angulaire (α) d'environ 15° vers l'arrière, par rapport à l'axe (XX') du trou médian (1b) (figure 5).

Plus généralement, quel que soit le nombre de broches (2) (3) et (4), dans le plan horizontal, les trous (1a) (1b) (1c) sont orientés angulairement pour que la broche (3) engagée dans le trou médian (1b), soit orientée en direction du centre de la tête fémorale, que la broche supérieure soit orientée vers l'avant de la tête fémorale et que la broche inférieure soit orientée vers l'arrière de la tête fémorale. Avantageusement, les broches (2) (3) et (4) sont engagées selon un mouvement de vrille, dans le sens horaire pour le fémur droit et dans le sens inverse, c'est-à-dire trigonométrique, pour le fémur gauche.

Les extrémités (2a) (3a) (4a) des broches (2) (3) (4) engagées dans la tête fémorale, peuvent être lisses ou filetées. De même, les parties (2b) (3b) (4b) des broches (2) (3) (4) engagées dans le clou (1), peuvent être lisses ou filetées pour y être vissées.

Les têtes (2b1) (3b1) (4b1) sont alignées très sensiblement verticalement sur la corticale externe du fémur. Les broches (2) (3) et (4) sont de longueur différente et croissante de haut en bas.

Enfin, de manière parfaitement connue, l'extrémité distale du clou peut être vissée dans la partie correspondante du fémur. De même, le clou peut présenter au niveau de sa partie distale, une section transversale nervurée (1d) (figure 6).

Le clou (1) est conformé pour coopérer avec un guide clou au moment de son impaction. La partie proximale du clou est pleine et agencée en (1e) pour permettre son impaction, le montage d'un appareil du type viseur, pour le perçage au moyen de douille, des trois trous (1a) (1b) (1c) et enfin le montage d'un appareil extracteur pour son ablation éventuelle.

Comme le montrent les figures 6 et 7, l'ostéosynthèse ainsi définie permet d'obtenir, d'une part, une très grande résistance à la compression par le fait que toutes les extrémités (2a) (3a) (4a) des broches sont dans le plan médian et, d'autre part, un système dynamisé par le fait que la tête fémorale peut coulisser sur les broches et venir mettre en compression le trait de fracture. De même, les broches sont convenablement réparties au niveau du col du fémur qui a une section réduite, évitant tout risque de fracture des corticales.

Les avantages ressortent bien de la description.

## Revendications

1. Dispositif d'enclouage centro-médullaire notamment pour fractures sous cervicales d'un fémur comprenant une tête fémorale, le dispositif étant du type comprenant un clou centro-médulaire (1) impacté dans le canal médulaire du fémur et une série d'au moins deux broches (2) (3) (4) introduites dans la corticale externe du fémur et engagées dans des trous (1a) (1b) (1c) du clou (1), caractérisé en ce que lesdits trous sont orientés angulairement dans un plan vertical et dans un plan horizontal pour que les extrémités des broches soient alignées sur le diamètre antéro-postérieur de la tête fémorale, ce qui correspond au plan de charge du patient, dans le plan horizontal, les trous (1a) (1b) (1c) sont orientés angulairement pour que la broche (3) engagée dans le trou médian (1b), soit orientée en direction du centre de la tête fémorale, que la broche supérieure (2) soit orientée vers l'avant de la tête fémorale et que la broche inférieure (4) soit orientée vers l'arrière de la tête fémorale.

2. Dispositif selon la revendication 1, caractérisé en ce que les extrémités des broches (2) (3) (4) sont régulièrement réparties sur toute la longueur du diamètre.

3. Dispositif selon la revendication 1, caractérisé en ce qu'en projection dans le plan frontal, les trous (1a) (1b) (1c) sont orientés angulairement pour que leurs axes convergent en direction du centre de la tête fémorale.

4. Dispositif selon la revendication 1, caractérisé en ce que l'axe du trou supérieur (1a) est décalé d'environ 15° vers l'avant, l'axe du trou inférieur (1c) étant décalé d'environ 15° vers l'arrière, tandis que l'axe ou les axes du ou des trous intermédiaires se répartissent entre lesdits axes des trous supérieur et inférieur.

5. Dispositif selon la revendication 1, caractérisé en ce que les extrémités des broches (2) (3) (4) engagées dans la tête fémorale sont filetées.

6. Dispositif selon la revendication 5, caractérisé en ce que les parties (2b) (3b) (4b) des broches engagées dans le clou (1) sont lisses.

7. Dispositif selon la revendication 5, caractérisé en ce que les parties des broches (2b) (3b) (4b) engagées dans le clou (1) sont filetées pour y être vissées.

8. Dispositif selon la revendication 5, caractérisé en ce que les têtes (2b1) (3b1) (4b1) des broches (2) (3) (4), sont alignées très sensiblement verticalement sur la corticale externe du fémur.

9. Dispositif selon la revendication 1, caractérisé en ce que les broches (2) (3) (4) sont de longueurs différentes et croissantes de haut en bas.

10. Dispositif selon la revendication 1, caractérisé en ce que la partie proximale du clou (1) recevant les broches (2) (3) (4) est pleine et présente des agencements (1e).

## Claims

1. Centromedullary nailing device, in particular for subcervical fractures of a thigh bone comprising a femoral head, the device being of the type comprising a centromedullary nail (1) driven into the medullary canal of the thigh bone and a series of at least two pins (2, 3, 4) inserted into the outer cortical substance of the thigh bone and engaged in holes (1a, 1b, 1c) in the nail (1), characterised in that said holes have an angular orientation in a vertical plane and in a horizontal plane so that the ends of the pins are aligned with the anteroposterior diameter of the femoral head, which corresponds to the load plane of the patient, in the horizontal plane, the holes (1a, 1b, 1c) have an angular orientation so that the pin (3) engaged in the central hole (1b) is directed towards the centre of the femoral head, that the upper pin (2) is directed towards the front of the femoral head and that the lower pin (4) is directed towards the rear of the femoral head.

2. Device according to Claim 1, characterised in that the ends of the pins (2, 3, 4) are uniformly distributed along the entire length of the diameter.

3. Device according to Claim 1, characterised in that in projection in the frontal plane, the holes (1a, 1b, 1c) have an angular orientation so that their axes converge in the direction of the centre of the femoral head.

4. Device according to Claim 1, characterised in that the axis of the upper hole (1a) is offset by approximately 15° towards the front, the axis of the lower hole (1c) being offset by approximately 15° towards the rear, whereas the axis or the axes of the intermediate hole or holes are distributed between said axes of the upper and lower holes.

5. Device according to Claim 1, characterised in that the ends of the pins (2, 3, 4) engaged in the femoral head are screw-threaded.

6. Device according to Claim 5, characterised in that the parts (2b, 3b, 4b) of the pins engaged in the nail (1) are smooth.

7. Device according to Claim 5, characterised in that the parts of the pins (2b, 3b, 4b) engaged in the nail (1) are screw-threaded in order to be screwed therein.

8. Device according to Claim 5, characterised in that the heads (2b1, 3b1, 4b1) of the pins (2, 3, 4) are aligned very substantially vertically with the outer cortical substance of the femur.

9. Device according to Claim 1, characterised in that the pins (2, 3, 4) have different lengths and increase from the top downwards.

10. Device according to Claim 1, characterised in that the proximal part of the nail (1) receiving the pins (2, 3, 4) is solid and has arrangements (1e).

## Patentansprüche

1. Centromedullär-Nagelungsvorrichtung, insbesondere für subcervicale Brüche eines Oberschenkels mit einem Oberschenkelkopf, wobei die Vorrichtung von einer Art ist, welche einen in den medullären Kanal des Oberschenkels geschlagenen centromedullären Nagel (1) und eine Reihe von wenigstens zwei in die externe Corticalis des Oberschenkels eingeführten und in Löcher (1a) (1b) (1c) des Nagels (1) eingesetzte Stifte (2) (3) (4) aufweist, dadurch gekennzeichnet, daß die Löcher in einer vertikalen Ebene und in einer horizontalen Ebene so orientiert sind, daß die Enden der Stifte auf den Vorne-Hinten-Durchmesser des Oberschenkelkopfes, was der Belastungsebene des Patienten entspricht, in der horizontalen Ebene ausgerichtet sind, wobei die Löcher (1a) (1b) (1c) winkelmäßig so orientiert sind, daß der in das mittlere Loch (1b) eingesetzte Stift (3) in Richtung Mitte des Oberschenkelkopfs orientiert ist, daß der obere Stift (2) zum Vorderteil des Oberschenkelkopfs orientiert ist und daß der untere Stift (4) zum hinteren Teil des Oberschenkelkopfs orientiert ist.

2. Vorrichtung nach Anspruch 1, dadurch gekennzeichnet, daß die Enden der Stifte (2) (3) (4) regelmäßig über die gesamte Länge des Durchmessers verteilt sind.

3. Vorrichtung nach Anspruch 1, dadurch gekennzeichnet, daß in Projektion in die Frontalebene die Löcher (1a) (1b) (1c) winkelmäßig so orientiert sind, daß ihre Achsen in Richtung Mitte des Oberschenkelkopfs zusammenlaufen.

4. Vorrichtung nach Anspruch 1, dadurch gekennzeichnet, daß die Achse des oberen Loches (1a) ungefähr 15° nach vorne versetzt ist, wobei die Achse des unteren Loches (1c) um ungefähr 15° nach hinten versetzt ist, während die Achse oder die Achsen des oder der Zwischenlöcher sich zwischen den Achsen des oberen und unteren Lochs verteilen.

5. Vorrichtung nach Anspruch 1, dadurch gekennzeichnet, daß die in den Oberschenkelkopf eingesetzten Enden der Stifte (2) (3) (4) mit einem Gewinde versehen sind.

6. Vorrichtung nach Anspruch 5, dadurch gekennzeichnet, daß die in den Nagel (1) eingesetzten Teile (2b) (3b) (4b) der Stifte glatt sind.

7. Vorrichtung nach Anspruch 5, dadurch gekennzeichnet, daß die in den Nagel (1) eingesetzten Teile der Stifte (2b) (3b) (4b) für eine Verschraubung mit diesem mit einem Gewinde versehen sind.

8. Vorrichtung nach Anspruch 5, dadurch gekennzeichnet, daß die Köpfe (2b1) (3b1) (4b1) der Stifte (2) (3) (4) sehr wesentlich senkrecht auf die externe Corticalis des Oberschenkels ausgerichtet sind.

9. Vorrichtung nach Anspruch 1, dadurch gekennzeichnet, daß die Stifte (2) (3) (4) unterschiedliche und von oben nach unten zunehmende Länge haben.

10. Vorrichtung nach Anspruch 1, dadurch gekennzeichnet, daß der die Stifte (2), (3), (4) aufnehmende proximale Teil des Nagels (1) voll ist und Einrichtungen (1e) aufweist.
